**Europäisches Patentamt**

**European Patent Office**

**Office européen des brevets**

(11) Veröffentlichungsnummer: **0 065 709**
**B1**

(19)

# (12) EUROPÄISCHE PATENTSCHRIFT

(45) Veröffentlichungstag der Patentschrift:
08.08.84

(51) Int. Cl.³: **C 07 C 87/18, C 07 C 85/26**

(21) Anmeldenummer: **82104159.7**

(22) Anmeldetag: **13.05.82**

(54) **Verfahren zur Reinigung von N,N-Dimethylaminopropylamin.**

(30) Priorität: **23.05.81 DE 3120558**

(43) Veröffentlichungstag der Anmeldung:
**01.12.82 Patentblatt 82/48**

(45) Bekanntmachung des Hinweises auf die Patenterteilung:
**08.08.84 Patentblatt 84/32**

(84) Benannte Vertragsstaaten:
**BE CH DE FR GB IT LI NL SE**

(56) Entgegenhaltungen:
**DE - A - 2 253 594**
**US - A - 3 038 904**

**J. AM. CHEM. SOC., Band 78, mai 1956, Seiten 1816-18, Washington D.C. (USA); G.B. HARES et al.: "Equilibrium constants for the formation of complexes between metal ions and polyamines"**

(73) Patentinhaber: **Ruhrchemie Aktiengesellschaft, Bruchstrasse 219, D-4200 Oberhausen 13 (DE)**

(72) Erfinder: **Cornils, Boy, Dr. Dipl.-Chem., Friedrich-Ebert-Strasse 45, D-4220 Dinslaken (DE)**
Erfinder: **Wiebus, Ernst, Ferdinandstrasse 77, D-4200 Oberhausen 14 (DE)**
Erfinder: **Breitkopf, Norbert, Am Nappenfeld 26, D-4250 Bottrop (DE)**

(74) Vertreter: **Reichelt, Karl-Heinz, Dr., m. Br. Ruhrchemie Aktiengesellschaft Abt. PLD Postfach 13 01 60, D-4200 Oberhausen 13 (DE)**

**Beschreibung**

N,N-Dimethylaminopropylamin (N,N-Dimethyl-1,3-diaminopropan, 3-Dimethylaminopropylamin) stellt ein wichtiges Zwischenprodukt zur großtechnischen Herstellung von Pharmazeutika, oberflächenaktiven Mitteln, Härtern für Epoxidharze oder Ionenaustauschern dar und wird auch als Vorprodukt zur Herstellung von Flockungsmitteln, Straßenmarkierungsfarben und Polyurethanen benötigt.

N,N-Dimethylaminopropylamin (DMAPA) wird üblicherweise durch Umsetzung von Acrylnitril und Dimethylamin hergestellt. Bei dieser Additionsreaktion bildet sich Dimethylaminopropionitril. Ein Verfahren dieser Art, nämlich Dimethylamin mit Acrylnitril in einem Blasensäulenreaktor umzusetzen, ist in der DE-PS 2 709 966 beschrieben.

Durch anschließende Hydrierung des Rohproduktes in der Gasphase oder Flüssigphase wird das N,N-Dimethylaminopropylamin erhalten. Als Hydrierkatalysatoren werden handelsübliche Trägerkatalysatoren auf der Basis von Nickel oder Kobalt verwendet. Im Verlauf dieser mehrstufigen Synthese bilden sich infolge von Konkurrenzumsetzungen und Folgereaktionen Nebenprodukte, die wieder mit den Einsatzprodukten reagieren können.

So finden sich in dem Rohgemisch nach Hydrierung des Dimethylaminopropionitrils neben dem erwünschten N,N-Dimethylaminopropylamin im wesentlichen folgende Produkte: Trimethylamin (3°C), n-Propylamin (47,8°C), Allylamin (58°C), N-Methyl-n-propylamin (62,5°C), N,N-Dimethyl-n-propylamin (65,6°C), Propionitril (97,4°C), Di-n-Propylamin (110°C), N,N,N'-Trimethyl-1,3-diaminopropan (138°C), N-Methyl-1,3-diaminopropan (140°C), N,N,N',N'-Tetramethyl-1,3-diaminopropan (145°C), Trin-Propylamin (156°C), N-Methyl-aminopropionitril (ca. 170°C), N,N-Dimethylaminopropionitril (177°C) und N,N,N',N'-Tetramethyl-dipropylentriamin (241°C).

Die Zahlenangaben in Klammern entsprechen jeweils dem Siedepunkt der entsprechenden Substanz bei 1013 mbar.

Das gewünschte Hauptprodukt N,N-Dimethylaminopropylamin besitzt einen Siedepunkt von 134,8°C (bei 1013 mbar).

Aufgrund der Vielzahl der zwangsweise entstehenden Nebenprodukte ist es unerläßlich, das erwünschte N,N-Dimethylaminopropylamin einer Reinigung zu unterziehen. Üblicherweise wird einer destillativen Aufarbeitung der Vorzug gegeben. Dabei zeigt sich, daß alle vorgenannten Komponenten sich mittels eines zwar aufwendigen, aber dennoch durchführbaren Destillationsverfahrens vollständig abtrennen lassen.

Allerdings gilt dies nicht für die ebenfalls im Rohgemisch enthaltene Komponente 1,3-Diaminopropan, deren Siedepunkt bei 136°C (gemessen bei 1013 mbar) liegt. Diese Verunreinigung, die in Abhängigkeit vom Alter des Hydrierkatalysators stets in einer Menge von etwa 200 bis ca. 5000 ppm im Rohgemisch enthalten ist, läßt sich selbst bei sehr aufwendig gestalteter Destillationsführung (hohe Bodenzahl und hohes Rücklaufverhältnis) nicht im gewünschten Maße entfernen. Eine Abtrennung von 1,3-Diaminopropan ist zwingend erforderlich, da seine Anwesenheit — selbst in geringsten Mengen — in der Nachverarbeitung zu nicht spezifikationsgerechten Zwischenprodukten oder unzulässigen Trübungen in Folgeprodukten führt.

Die geringfügige Differenz der Siedepunkte von 1,3-Diaminopropan und N,N-Dimethylaminopropylamin von lediglich etwa 2°C bei Normaldruck vergrößert sich zwar bei einer Destillation im Vakuum auf ca. 9°C bei 10 mbar, dennoch reicht selbst diese Siededifferenz nicht aus, 1,3-Diaminopropan so weit abzutrennen, daß das N,N-Dimethylaminopropylamin nur noch Restgehalte von unter 100 ppm an 1,3-Diaminopropan (bezogen auf N,N-Dimethylaminopropylamin) aufweist (siehe Vergleichsversuch 1).

Ein anderer, technisch häufig beschrittener Weg, Verunreinigungen aus einem Rohprodukt zu entfernen, besteht darin, die Verunreinigungen durch Zugabe geeigneter Azeotropbildner unter Ausnutzung sich ausbildender Azeotrope destillativ abzutrennen und so eine Reinigung herbeizuführen.

Aus der Literatur sind entsprechende Azeotrop-Daten für die dem 1,3-Diaminopropan formal ähnliche Verbindung 1,2-Diaminopropan bekannt (L. H. Horsley, Azeotropic data, Advances in Chemistry Series No. 116, American Chemical Society [Wash. D. C], 1973). Danach bilden sich Azeotrope aus 1,2-Diaminopropan mit i-Butanol oder Toluol aus. Ihre Siedepunkte werden mit 123°C bzw. 105°C angegeben. Die Gehalte an 1,2-Diaminopropan betragen 65 Gew.-% bzw. 32 Gew.-%. Unter der Voraussetzung, daß 1,3-Diaminopropan ein dem 1,2-Diaminopropan ähnliches Verhalten zeigt, würden die zu erwartenden Siedepunkterniedrigungen im Vergleich zu dem Siedepunkt von N,N-Dimethylaminopropylamin von 134,8°C bei geeigneten apparativen Voraussetzungen ausreichend sein, eine destillative Abtrennung vorzunehmen.

Wie Versuche zeigen, ist dieser Weg zur Abtrennung von 1,3-Diaminopropan ebenfalls nicht durchführbar. Die Restgehalte an 1,3-Diaminopropan liegen stets weit über der Grenze, die für hochreines N,N-Dimethylaminopropylamin als zulässig angesehen wird (siehe Vergleichsversuch 2, Tabelle 2 und 3).

Die US-A-3 038 904 betrifft ein Verfahren zur Trennung von Polyaminen. Unter Zusatz von Salzen des Kupfers, Zinks, Nickels und Kobalts lassen sich Gemische, bestehend aus Ethylendiamin bzw. Ethylendiaminderivaten und Piperazinderivaten, voneinander trennen. Die Ethylendiaminderivate bilden mit den Übergangsmetallsalzen Chelatverbindungen, aus denen die Piperazinderivate mittels Extraktion mit Chloroform gewonnen werden können. Bei Vorliegen wäßriger Polyaminlösungen scheiden sich die Chelatverbindungen als Niederschlag ab und können durch Filtration entfernt werden. Durch Rückspaltung der Chelatkomplexe mittels starker Basen werden die Ethylendiaminderivate freigesetzt. Das Verfahren eignet sich zur Präparation von Piperazinderivaten, die erhebliche Mengen an Ethylendiaminprodukten aufweisen.

Es bestand daher die Aufgabe, ein Verfahren zur Reinigung von N,N-Dimethylaminopropylamin bereitzustellen, das den Restgehalt an 1,3-Diaminopropan auf Werte von unter 100 ppm absenkt.

Überraschenderweise wird diese Aufgabe gelöst durch ein Verfahren zur Reinigung von 1,3-Diaminopropan als Verunreinigung enthaltendem N,N-Dimethylaminopropylamin, das durch Addition von Dimethylamin an Acrylnitril, anschließende Hydrierung und Destillation erhalten wird, dadurch gekennzeichnet, daß vor oder während der Destillation dem zu reinigenden Produkt Metallsalze der Übergangselemente des periodischen Systems zugesetzt werden. Unter Übergangselemente werden die Übergangsmetalle der Ordnungszahlen 21 bis 80 verstanden.

Diese Metallverbindungen können in Form anorganischer Mineralsalze, wie Nitrate, Chloride, Sulfate, Carbonate, in reiner Form oder als wäßrige Lösung eingesetzt werden. Darüber hinaus ist es ebenfalls möglich organometallische Verbindungen, wie Carbonyle, Acetylacetonate oder Carbonylate zu verwenden.

Die üblicherweise eingesetzten Mengen an Metallverbindungen betragen 0,5 bis 20 Mol pro Mol 1,3-Diaminopropan.

Aus den eingesetzten Metallverbindungen und der Verunreinigung 1,3-Diaminopropan bilden sich Metall-Chelate. Sie erweisen sich trotz der recht drastischen Bedingungen, wie sie in einer Destillation vorherrschen, als derart temperaturstabil, daß die einmal gebildeten Komplexe praktisch keine Neigung aufweisen, in ihre Ausgangskomponenten zu spalten. Nach diesem Verfahren gelingt es in sehr einfacher Weise den Gehalt des als Kopfprodukt der Destillation anfallenden N,N-Dimethylaminopropylamins an 1,3-Diaminopropan auf Werte von unter 100 ppm zu verringern.

Als Metalle kommen insbesondere in Betracht: Kobalt, Nickel, Chrom, Eisen, Mangan, Kupfer, Cadmium sowie Zink oder Quecksilber (siehe Beispiel 1).

Die neue, erfindungsgemäße Verfahrensgestaltung erweist sich im praktischen Betrieb als vorteilhaft und ermöglicht insbesondere die Reinigung bei geringem Energieaufwand für die Destillation infolge niedriger Rücklaufverhältnisse.

Zum einen kann das Metallsalz in Form von Tabletten in einem Festbettreaktor angeordnet werden, durch das das Rohprodukt vor Einsatz in die Destillation strömt, zum anderen ist es möglich, eine wäßrige Metallsalzlösung direkt in den Sumpf einer Destillationskolonne einzuspeisen, um den gewünschten Reinigungseffekt zu erzielen. Beide Möglichkeiten werden durch die nachfolgenden Beispiele 2 und 3 belegt.

Besonders geeignet — und wegen des vergleichsweise niedrigen Preises auch wirtschaftlich interessant — ist der Einsatz von Nickelverbindungen, insbesondere Nickelsulfat.


Vergleichsversuch 1


2000 g N,N-Dimethylaminopropylamin werden einer fraktionierten, diskontinuierlichen Destillation unterworfen. Die verwendete Kolonne weist 80 theoretische Böden auf (Füllkörperkolonne). Es werden Fraktionen von jeweils 10 Gew.-% abgezogen und auf ihren Gehalt an 1,3-Diaminopropan untersucht. Der Gehalt an 1,3-Diaminopropan im Einsatzprodukt (N,N-Dimethylaminopropylamin-Rohprodukt) beträgt 1400 ppm.

Das Rücklaufverhältnis wird auf 20 Teile Rücklauf pro Teil abgenommenen Kopfprodukts eingestellt. Die Bestimmung des Gehaltes an 1,3-Diaminopropan erfolgt durch gaschromatographische Analyse.

Wie aus Tabelle 1 ersichtlich, findet keine signifikante Anreicherung von 1,3-Diaminopropan in den Nachlauffraktionen, wie aufgrund des höheren Siedepunktes zu erwarten wäre, statt.

3

Tabelle 1

| Fraktion | Temperatur des Kopfproduktes | Temperatur des Sumpfes | Rücklauf-verhältnis | Druck (mbar) | Gehalt an 1,3-Diaminopropan in ppm |
|---|---|---|---|---|---|
| 1 | 133 | 136 | 20 : 1 | 1013 | 1500 |
| 2 | 134 | 137 | 20 : 1 | 1013 | 1400 |
| 3 | 134 | 138 | 20 : 1 | 1013 | 1800 |
| 4 | 134 | 137 | 20 : 1 | 1013 | 1400 |
| 5 | 134 | 138 | 20 : 1 | 1013 | 1400 |
| 6 | 134 | 137 | 20 : 1 | 1013 | 1300 |
| 7 | 134 | 137 | 20 : 1 | 1013 | 1400 |
| 8 | 134 | 140 | 20 : 1 | 1013 | 1400 |
| 9 | 134 | 140 | 20 : 1 | 1013 | 1400 |
| 10 | 134 | 140 | 20 : 1 | 1013 | 1400 |

## Vergleichsversuch 2

500 g N,N-Dimethylaminopropylamin werden mit 750 g Toluol bzw. 750 g i-Butanol versetzt und einer fraktionierten diskontinuierlichen Destillation unterworfen. Die verwendete Laborkolonne weist 40 theoretische Böden auf (Füllkörperkolonne). Es werden Fraktionen von 10 Gew.-% abgezogen und auf den Gehalt an 1,3-Diaminopropan untersucht. Der Gehalt an 1,3-Diaminopropan im Einsatzprodukt (N,N-Dimethylaminopropylamin-Rohprodukt) beträgt 1400 ppm.

Das Rücklaufverhältnis wird auf 10 Teile Rücklauf pro Teil abgenommenen Kopfprodukts eingestellt. Die Bestimmung des Gehaltes an 1,3-Diaminopropan erfolgt durch gaschromatographische Analyse.

Wie aus Tabelle 2 und 3 ersichtlich, findet keine signifikante Anreicherung von 1,3-Diaminopropan als Azeotrop mit dem Vorlauf aus Toluol bzw. i-Butanol statt; die nach Abtrennung der Vorlauffraktionen übergehende Hauptfraktion aus N,N-Dimethylaminopropylamin enthält weiterhin ca. 1400 ppm 1,3-Diaminopropan.

Tabelle 2 gibt die Ergebnisse unter Zusatz von Toluol, Tabelle 3 die Resultate unter Verwendung von i-Butanol wieder.

Tabelle 2 (Toluol-Zusatz)

| Fraktion | Kopf-temperatur | Sumpf-temperatur | Rücklauf-verhältnis | Druck (mbar) | Gehalt an 1,3-Diaminopro-pan in ppm |
|---|---|---|---|---|---|
| 1*) | 111 | 122 | 10 : 1 | 1013 | 50 |
| 2*) | 111 | 124 | 10 : 1 | 1013 | 50 |
| 3*) | 111 | 124 | 10 : 1 | 1013 | 50 |
| 4*) | 111 | 129 | 10 : 1 | 1013 | 50 |
| 5*) | 112 | 134 | 10 : 1 | 1013 | 50 |
| 6**) | 135 | 137 | 10 : 1 | 1013 | 700 |
| 7 | 135 | 138 | 10 : 1 | 1013 | 1300 |
| 8 | 135 | 138 | 10 : 1 | 1013 | 1400 |
| 9 | 135 | 160 | 10 : 1 | 1013 | 1300 |
| 10 | 133 | 220 | 10 : 1 | 1013 | 1000 |

\*) Toluolgehalt > 99%
\*\*) Toluolgehalt ca. 50%

Tabelle 3 (i-Butanol-Zusatz)

| Fraktion | Kopf-temperatur | Sumpf-temperatur | Rücklauf-verhältnis | Druck (mbar) | Gehalt an 1,3-Diaminopro-pan in ppm |
|---|---|---|---|---|---|
| 1*) | 108 | 110 | 10 : 1 | 1013 | < 100 |
| 2*) | 108 | 113 | 10 : 1 | 1013 | < 100 |
| 3*) | 108 | 117 | 10 : 1 | 1013 | < 100 |
| 4*) | 108 | 117 | 10 : 1 | 1013 | < 100 |
| 5*) | 108 | 128 | 10 : 1 | 1013 | < 100 |
| 6*) | 132 | 139 | 10 : 1 | 1013 | < 100 |
| 7 | 134 | 140 | 10 : 1 | 1013 | 1500 |
| 8 | 135 | 140 | 10 : 1 | 1013 | 1300 |
| 9 | 135 | 140 | 10 : 1 | 1013 | 1500 |
| 10 | 134 | 180 | 10 : 1 | 1013 | 1200 |

\*) Isobutanolgehalt > 99%

## Beispiel 1

800 g N,N-Dimethylaminopropylamin-Rohprodukt mit einem Gehalt von 220 ppm 1,3-Diaminopropan werden in einen 2 l Dreihalskolben, der mit Rückflußkühler, Rührer und Innenthermometer ausgestattet ist, eingefüllt und je Versuch mit 1 Gew.-% Kupfercarbonat oder Eisen(III)-sulfat oder basischem Chromsulfat oder Kobaltcarbonat versetzt, unter intensivem Rühren auf 70°C aufgewärmt und 6 Std. bei dieser Temperatur gerührt. Anschließend wird das N,N-Dimethylaminopropylamin durch Abflashung von überschüssigem Metallsalz und vom Chelatkomplex befreit. Der Gehalt des abgeflashten N,N-Dimethylaminopropylamins an 1,3-Diaminopropan ist der folgenden Tabelle 4 zu entnehmen. Die weitere Reinigung des Rohproduktes erfolgt durch fraktionierte Destillation.

Tabelle 4

| Eingesetztes Salz | Gehalt an 1,3-Diaminopropan (ppm) |
|---|---|
| Kupfercarbonat | 80 |
| Eisen(III)-sulfat | 70 |
| Bas. Chromsulfat | 95 |
| Kobalt(II)-carbonat | 65 |

## Beispiel 2

Ein zylindrisches Glasgefäß mit einem Volumen von 500 ml wird mit Nickelsulfat-Pellets gefüllt. Durch Außenheizung wird es auf 120°C aufgeheizt und diese Temperatur konstant gehalten. Pro Stunde werden durch diese Festbett-Schüttung von unten nach oben strömend 100 ml N,N-Dimethylaminopropylamin, das einen Gehalt von 1400 ppm an 1,3-Diaminopropan aufweist, durchgesetzt.

Das am Kopf des Reaktionsrohres austretende N,N-Dimethylaminopropylamin weist einen Gehalt von ca. 80 ppm an 1,3-Diaminopropan auf. Die weitere Reinigung erfolgt durch fraktionierte Destillation.

## Beispiel 3

Bei kontinuierlicher, destillativer Aufarbeitung von N,N-Dimethylaminopropylamin-Rohprodukt wird während der Abtrennung der niedrig siedenden Vorlaufkomponenten dem Sumpf der Kolonne Nickelsulfat ($NiSO_4 \cdot 7 H_2O$) in Form einer 25%igen wäßrigen Lösung zugesetzt. Pro Mol 1,3-Diaminopropan werden 1,5 Mol Nickelsulfat zugegeben. Die Sumpftemperatur liegt bei ca. 140°C, und die mittlere Verweilzeit des Produktgemisches im Kolonnensumpf beträgt ca. 1 Stunde.

Der Zusatz des Nickelsulfates bewirkt eine Abnahme des 1,3-Diaminopropan-Gehaltes von ursprünglich ca. 300 ppm auf einen Restanteil von nur ca. 30 ppm.

**Patentansprüche**

1. Verfahren zur Reinigung von 1,3-Diaminopropan als Verunreinigungen enthaltendem N,N-Dimethylaminopropylamin, das durch Addition von Dimethylamin an Acrylnitril, anschließende Hydrierung und Destillation erhalten wird, dadurch gekennzeichnet, daß vor oder während der Destillation dem zu reinigenden Produkt Metallsalze der Übergangselemente des periodischen Systems zugesetzt werden.

2. Verfahren nach Anspruch 1, dadurch gekennzeichnet, daß die Metallsalze der Übergangselemente in Form von Chloriden, Sulfaten, Carbonaten oder als organometallische Verbindungen, wie Carbonyle, Acetylacetonate oder Carbonylate eingesetzt werden.

3. Verfahren nach Anspruch 1 und 2, dadurch gekennzeichnet, daß pro Mol 1,3-Diaminopropan 0,5 bis 20 Mol an Metallverbindung verwendet werden.

4. Verfahren nach Anspruch 1 bis 3, dadurch gekennzeichnet, daß Salze der Übergangselemente Kobalt, Nickel, Chrom, Eisen, Mangan, Kupfer, Cadmium, Zink oder Quecksilber verwendet werden.

5. Verfahren nach Anspruch 1 bis 4, dadurch gekennzeichnet, daß das Metallsalz des Übergangselementes in Form eines Festbettes angeordnet ist.

6. Verfahren nach Anspruch 1 bis 4, dadurch gekennzeichnet, daß das Metallsalz in Form einer wäßrigen Lösung direkt in den Sumpf einer Destillationskolonne eingespeist wird.

7. Verfahren nach Anspruch 1 bis 6, dadurch gekennzeichnet, daß als Metallsalze Nickelsulfat verwendet wird.

## Claims

1. Method for purifying N,N-dimethylaminopropylamine, obtained by adding dimethylamine to acrylonitrile followed by hydrogenation and distillation, and containing 1,3-diaminopropane as impurity, characterised in that metal salts of the transition elements of the Periodic System are added before or during the distillation to the product to be purified.

2. Method according to claim 1, characterised in that the metal salts of the transition elements are used in the formof chlorides, sulphates, carbonates or as organometallic compounds, such as carbonyls, acetyl acetonates, or carbonylates.

3. Method according to claims 1 and 2, characterised in that 0.5 to 20 moles of metal compound is used per mole of 1,3-diaminopropane.

4. Method according to claims 1 to 3, characterised in that salts of the transition elements cobalt, nickel, chromium, iron, manganese, copper, cadmium, zinc or mercury are used.

5. Method according to claims 1 to 4, characterised in that the metal salt of the transition element is arranged in the form of a fixed bed.

6. Method according to claims 1 to 4, characterised in that the metal salt is fed in the form of an aqueous solution directly to the bottom of a distillation column.

7. Method according to claims 1 to 6, characterised in that nickel sulphate is used as metal salt.

## Revendications

1. Procédé pour la N,N-diméthylaminopropylamine contenant en impureté le 1,3-diaminopropane et qui a été obtenue par addition de la diméthylamine sur l'acrylonitrile suivie d'une hydrogénation et d'une distillation, caractérisé en ce que, avant ou durant la distillation, on ajoute au produit à purifier des sels métalliques des éléments de transition de la Classification Périodique.

2. Procédé selon la revendication 1, caractérisé en ce que les sels métalliques des éléments de transition sont mis en œuvre à l'état de chlorures, sulfates, carbonates ou à l'état de composés organo-métalliques tels que des carbonyles, des acétylacétonates ou des carbonylates.

3. Procédé selon les revendications 1 et 2, caractérisé en ce que l'on utilise de 0,5 à 20 moles de composé métallique par mole du 1,3-diaminopropane.

4. Procédé selon les revendications 1 à 3, caractérisé en ce que l'on utilise les sels des éléments de transition cobalt, nickel, chrome, fer, manganèse, cuivre, cadmium, zinc ou mercure.

5. Procédé selon les revendications 1 à 4, caractérisé en ce que le sel métallique de l'élément de transition est disposé à l'état de lit fixe.

6. Procédé selon les revendications 1 à 4, caractérisé en ce que le sel métallique est introduit directement, à l'état de solution aqueuse, dans le pied d'une colonne à distiller.

7. Procédé selon les revendications 1 à 6, caractérisé en ce que le sel métallique utilisé est le sulfate de nickel.